**Europäisches Patentamt**

**European Patent Office**

**Office européen des brevets**

(11) Publication number: **0 195 114**

**B1**

# EUROPEAN PATENT SPECIFICATION

(45) Date of publication of patent specification: **16.05.90**

(21) Application number: **85109621.4**

(22) Date of filing: **31.07.85**

(51) Int. Cl.⁵: **A 61 K 31/71**, A 61 K 9/08, A 61 K 9/00, A 61 K 47/00, A 61 K 31/70

(54) **Stabilized injection compositions and their preparations.**

(30) Priority: **07.03.85 HU 85985**

(43) Date of publication of application:
**24.09.86 Bulletin 86/39**

(45) Publication of the grant of the patent:
**16.05.90 Bulletin 90/20**

(84) Designated Contracting States:
**CH DE FR LI**

(56) References cited:
**EP-A-0 003 150**
**DE-A-2 726 208**
**GB-A-1 470 676**

(73) Proprietor: **CHINOIN Gyogyszer és Vegyészeti Termékek Gyára RT.**
**To utca 1-5**
**H-1045 Budapest IV (HU)**

(72) Inventor: **Kálóy, Katalin, Dr.**
**22, Bertalan L.u.**
**Budapest 1111 (HU)**
Inventor: **Somfai, Eva, Dr.**
**8, Táncsics M.u.**
**Budapest 1014 (HU)**
Inventor: **Járay, Miklós, Dr.**
**20, Alom u.**
**Budapest 1125 (HU)**
Inventor: **Kovács, Enikö**
**4, Róna park**
**Budapest 1142 (HU)**

(74) Representative: **Patentanwälte Beetz sen. - Beetz jun. Timpe - Siegfried - Schmitt-Fumian**
**Steinsdorfstrasse 10**
**D-8000 München 22 (DE)**

Courier Press, Leamington Spa, England.

## EP 0 195 114 B1

**Description**

The invention relates to pH and colour stabilized, aqueous parenteral pharmaceutical compositions comprising an antibacterial aminoglycoside comprising a pyranose ring being unsaturated between the positions 4' and 5' and substituted with an aminoalkyl group at position 5', preferably sisomicine, netylmicine or 5-epi-sisomicine, and the parenterally acceptable salts thereof, and to a process for the preparation of these compositions.

It is known from the art that certain problems arise in connection with the stabilization of the colour and the pH of solutions of antibiotics belonging to the above group. In order to overcome the problems it was suggested (Hungarian patent application No. SchE-699 and EP—A—0003150) to adjust the starting pH of the composition to 5,0—6,5, and preferably, but not necessarily, to add an antioxidant. According to this specification a stable composition can preferably be prepared at a pH of 6,2 to 6,5.

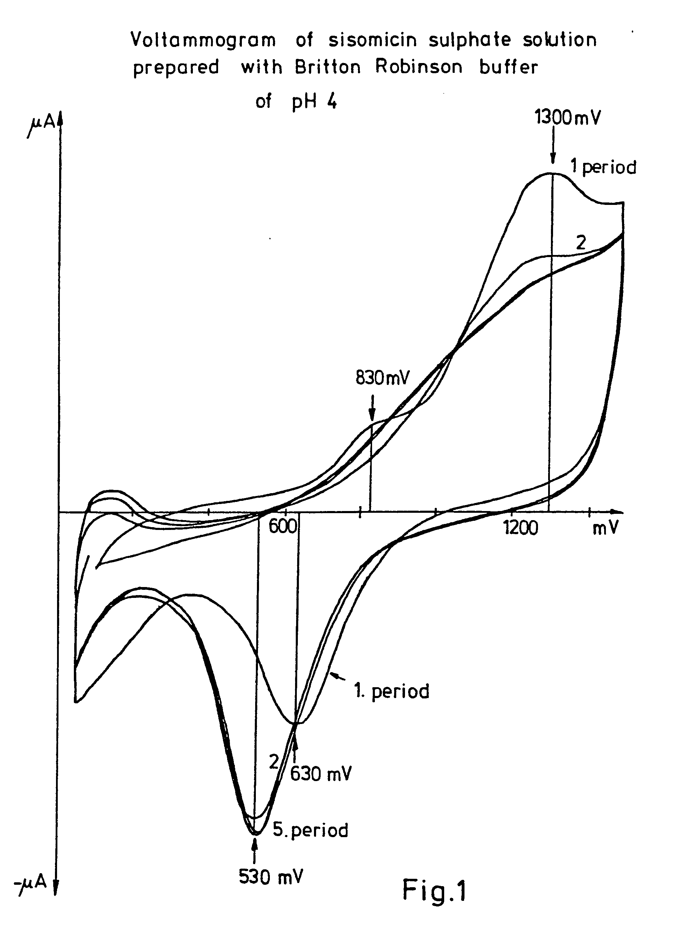

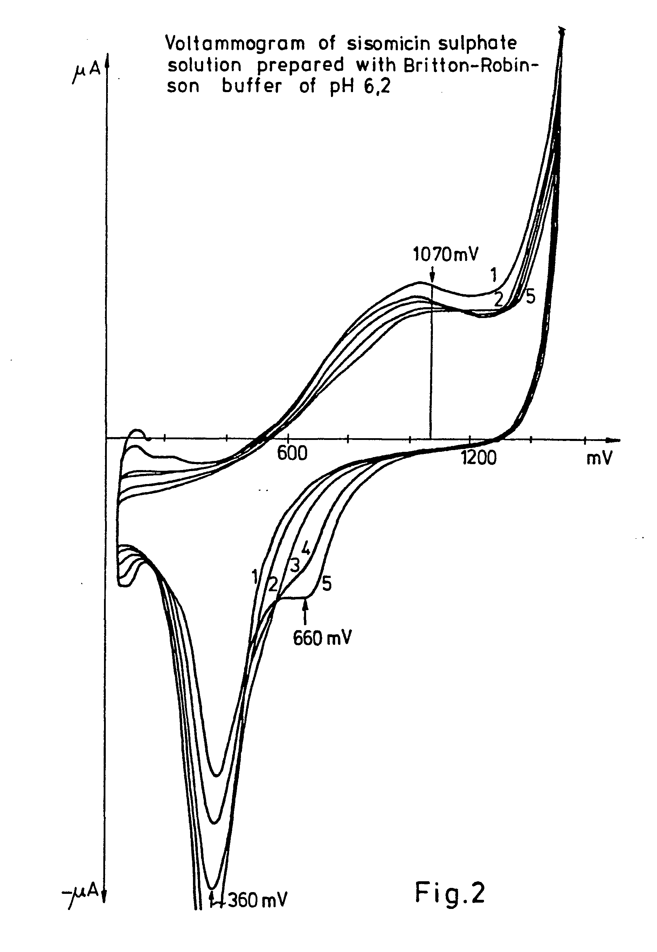

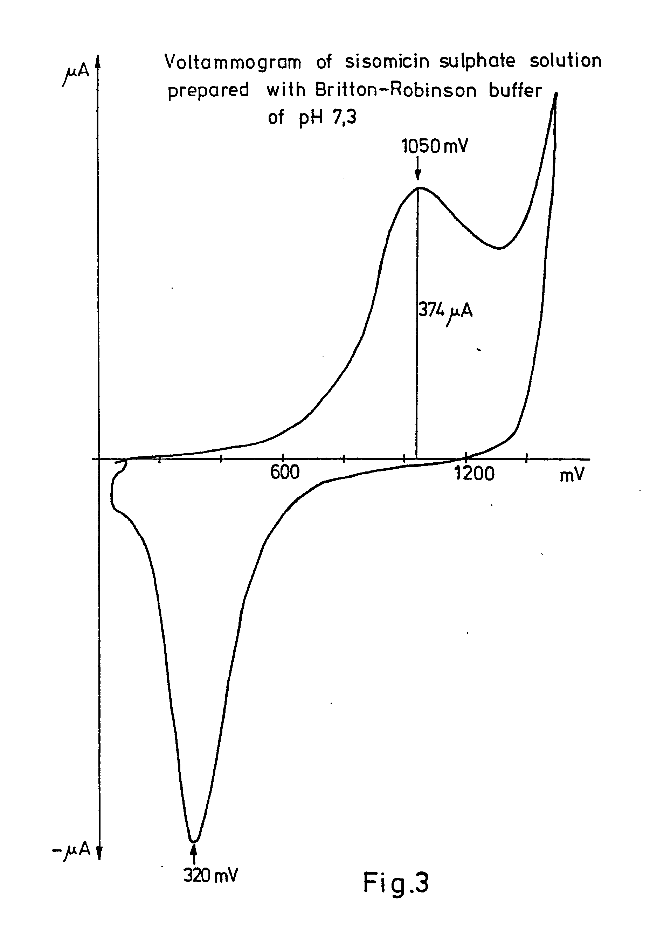

Upon examining the electrochemical oxidation of sisomicine sulphate at different pH values it was found that the peak potential of the electrochemical oxidation decreases to a certain limit with increasing pH. The Figures 1, 2 and 3 show the voltametric curves obtained by electrochemical oxidation of formulations having the same composition as described in Example 1, comprising sisomicine sulphate as active ingredient.

The curves show that a lower peak potential belongs to higher pH values. It has surprisingly been found that though the peak poitentials decrease with increasing pH values the sisomicine compositions adjusted to a higher pH value (pH 7,3) are more stable than the compositions having a lower pH value. This also applies for the compositions comprising the above-listed antibiotics.

Accordingly, stable pharmaceutical formulations can be prepared according to the invention from the above-listed antibiotics in a substantially higher pH range than was earlier described.

The pH of normal blood or plasma, i.e. of the physiological blood and tissues, is within the range of 7,3 to 7,4 and the same pH is applied according to the invention. Thus the antibiotics can be administered to the organism at an optimum pH, i.e. at the pH of the physiological blood. Therefore the treated organism is unaffected by adverse effects which is optimal from the point of view of medical treatment.

According to the invention stable pharmaceutical formulations comprising as active ingredient either sisomicine (Hungarian patent specification No. 079 145), netylmicine (Hungarian patent specification No. 170 513) or similar or related derivatives thereof can be prepared.

The pH and colour stabilized, aqueous parenteral pharmaceutical compositions according to the invention comprise an antibacterial aminoglycoside comprising a pyranose ring being unsaturated between positions 4' and 5' are substituted with an aminoalkyl group at position 5', are obtainable by
—dissolving the antibacterial aminoglycoside or a salt thereof in water,
—adjusting the pH of the solution to 7,3—7,4,
—adding an antioxidant to the solution,
—bubbling nitrogen through the solution after sterile filtration and
—filling the solution thus obtained into ampoules under nitrogen.

They comprise preferably sisomicine, netylmicine or 5-epi-sisomicine or the parenterally acceptable salts thereof as antibacterial aminoglycosides and are particularly suited for injections.

The process according to the invention for preparing the above defined pH and colour stabilized, aqueous parenteral pharmaceutical compositions is characterized by
—dissolving an antibacterial aminoglycoside comprising a pyranose ring being unsaturated between positions 4' and 5' and substituted with an aminoalkyl group at position 5' or a pharmacologically acceptable salt thereof in water,
—adjusting the pH of the solution to 7,3—7,4,
—adding an antioxidant to the solution,
—bubbling nitrogen through the solution after sterile filtration and
—filling the solution thus obtained into ampoules under nitrogen.

Preferably, sisomicine, netylmicine or 5-epi-sisomicine or the parenterally acceptable salts thereof are used as antibacterial aminoglycosides.

Phosphite or sulphite salts can preferably be used as antioxidants, but any other system may also be used which is neutral or not harmful to the organism.

The pH can be adjusted by addition of sodium hydroxide and/or buffer systems. 2-Amino-2-hydroxymethyl-1,3-propanediol buffer and/or sodium hydroxide+potassium hydrogenphosphate buffer can preferably be used.

The invention is illustrated in the following with reference to examples.

Example 1

A comparative experiment was carried out, wherein
—a sisomicine sulphate solution obtained without adjusting the pH (solution 1a)
—a solution described in Hungarian patent application No. SchE-699 and EP—A—0003150 at pH=6,2 which pH is declared to be the best by the aforementioned application (solution 1b) and
—the solution prepared according to the invention (solution 1c)

were compared from the point of view of stability.

The formulations of the compositions and their preparation were as follows:

Solution 1a:

|  | (mg) |
| --- | --- |
| Sisomicine (in the form of sisomicine sulphate) | 50,0 |
| Sodium metabisulphite | 3,0 |
| Methyl parabene | 0,8 |
| Propyl parabene | 0,1 |
| Disodium ethylenediamine tetraacetate | 0,1 |
| Sodium chloride | 3,6 |
| Distilled water | ad 1,0 ml. |

Solution 1b:

|  | (mg) |
| --- | --- |
| Sisomicine (in the form of sisomicine sulphate) | 50,0 |
| Sodium sulphite | 0,8 |
| Sodium metabisulphite | 2,4 |
| Propyl parabene | 0,1 |
| Methyl parabene | 0,8 |
| Disodium ethylenediamine tetraacetate | 0,1 |
| Sodium chloride | 3,9 mg |
| Distilled water | ad 1,0 ml. |

The pH of the solution was about 5,2. 0,1 N sodium hydroxide was added to the solution to adjust the pH to 6,2.

Solution 1c:

|  | (mg) |
| --- | --- |
| Sisomicine (in the form of sisomicine sulphate) | 50,0 |
| Sodium sulphite | 0,8 |
| Sodium metabisulphite | 2,4 |
| Propyl parabene | 0,1 |
| Methyl parabene | 0,8 |
| Disodium ethylenediamine tetraacetate | 0,1 |
| Sodium chloride | 3,9 |
| Distilled water | ad 1,0 ml. |

The pH of the solution was about 5,2. 1 N sodium hydroxide was added to the solution to adjust the pH to 7,35. The above solutions were prepared and filled under nitrogen atmosphere.

They were examined as follows:

The samples were treated at 40, 50 and 60°C in a thermostatized hot air chamber for 30 days; thereafter the pH, the colour and the active ingredient content were checked. The colour was compared with the colour measure according to pH. Hg. VI., the active ingredient content was determined microbiologically by measuring the biological activity. The results are summarized in the following table.

|  | Solution 1a 40°C | 50°C | 60°C | Solution 1b 40°C | 50°C | 60°C | Solution 1c 40°C | 50°C | 60°C |
| --- | --- | --- | --- | --- | --- | --- | --- | --- | --- |
| pH | 2,25 | 2,38 | 2,38 | 3,39 | 3,39 | 2,42 | 7,30 | 7,30 | 7,30 |
| colour | $S_5$ | $S_{6-7}$ | $S_{6-7}$ | $S_4$ | $S_{6-7}$ | $S_{6-7}$ | $S_0$—$S_1$ | $S_2$ | $S_2$ |
| active ingredient content (%) | 93,9 | 90,6 | 79,0 | 99,0 | 99,5 | 99,0 | 99,7 | 99,7 | 99,6 |

It could be observed that solution 1a was not resistent to the heat treatment. The active ingredient content of solution 1b was satisfactory after the heat treament, but its colour was strongly darkened even after the heat treatment at 40°C, and its pH significantly decreased. In the case of solution 1c according to the invention both the colour and the active ingredient content were suitable even after the heat treatment at 60°C, and the pH remained unchanged.

Example 2
A composition of the following formulation was prepared:

|  | (mg) |
|---|---|
| Sisomicine base (in the form of sulphate) | 10,0 |
| Propyl p-oxybenzoate (Ph. Hg. VI.) | 0,2 |
| Methyl p-oxybenzoate (Ph. Hg. VI.) | 1,3 |
| Disodium ethylenediamine tetraacetate (BP .73) | 0,1 |
| Sodium sulphite (BP. 80) | 1,5 |
| Sodium pyrosulphite (Ph. Hg. VI.) | 2,5 |
| Sodium chloride (Ph. Hg. VI.) | 4,9 |
| Distilled water suitable for injections (Ph. Hg. VI.) | ad 1,0 ml. |

The pH of the solution was 5,95. The pH of the solution was adjusted to 7,4 by adding 2-amino-2-hydroxymethyl-1,3-propanediol buffer. The solution was prepared by bubbling nitrogen gas through it and filtering thus obtained solution on a bacteria filter under sterile conditions and was filled into ampoules under nitrogen atmosphere.

The colour, pH and active ingredient stability of the solution were equivalent to those of the solution 1c of Example 1.

Example 3
A composition of the following formulation was prepared:

|  | (mg) |
|---|---|
| Netylmicine base (in the form of sulphate) | 50,0 |
| Propyl p-oxybenzoate (Ph. Hg. VI.) | 0,1 |
| Methyl p-oxybenzoate (Ph. Hg. VI.) | 0,8 |
| Disodium ethylenediamine tetraacetate (BP. 73) | 0,1 |
| Sodium pyrosulphite (Ph. Hg. VI.) | 3,0 |
| Sodium sulphite (PB. 80) | 4,0 |
| Sodium chloride (Ph. Hg. VI.) | 2,0 |
| Distilled water suitable for injections | ad 1,0 ml. |

The original pH of the solution was 6,2. It was adjusted to 7,3 by adding sodium hydroxide+potassium hydrogensulphate buffer system. The compositions was prepared and filled into ampoules according to Example 2.

The colour, pH and active ingredient stability of the solution were examined for 30 days at 40, 50 and 60°C, and were found to be satisfactory.

**Claims**

1. pH and colour stabilized, aqueous parenteral pharmaceutical compositions comprising an antibacterial aminoglycoside comprising a pyranose ring being unsaturated between positions 4' and 5' and substituted with an aminoalkyl group at positions 5', obtainable by
—dissolving the antibacterial aminoglycoside or a salt thereof in water
—adjusting the pH of the solution to 7,3—7,4,
—adding an antioxidant to the solution,
—bubbling nitrogen through the solution after sterile filtration and
—filling the solution thus obtained into ampoules under nitrogen.

2. Pharmaceutical compositions according to claim 1, obtainable by using sisomicine, nethylmicine or 5-epi-sisomicine or the parenterally acceptable salts thereof as the antibacterial aminoglycoside.

3. Pharmaceutical compositions according to claim 1 or 2, obtainable by using sodium phosphite or sodium sulphite as said antioxidant.

4. Pharmaceutical compositions according to one of claims 1 to 3, obtainable by adjusting the pH of the solution with a buffer system comprising 2-amino-2-hydroxymethyl-1,3-propanediol.

5. A process for preparing the pH and colour stabilized, aqueous parenteral pharmaceutical compositions according to one of claims 1 to 4, characterized by

4

—dissolving an antibacterial aminoglycoside comprising a pyranose ring being unsaturated between positions 4' and 5' and substituted with an aminoalkyl group at position 5' or a pharmacologically acceptable salt thereof in water,
—adjusting the pH of the solution to 7,3—7,4,
—adding an antioxidant to the solution,
—bubbling nitrogen through the solution after sterile filtration and
—filling the solution thus obtained into ampoules under nitrogen.

6. A process according to claim 5, characterized by using sisomicine, netylmicine or 5-epi-sisomicine or the parenterally acceptable salt thereof as the antibacterial aminoglycoside.

7. A process according to claim 5 or 6, characterized by using sodium phosphite or sodium sulphite as the antioxidant.

8. A process according to one of claims 5 to 7, characterized by adjusting the pH of the solution with a buffer system comprising 2-amino-2-hydroxymethyl-1,3-propanediol.

**Patentansprüche**

1. pH- und farbstabilisierte wässrige parenterale pharmazeutische Zusammensetzungen aus einem antibackteriellen Aminoglycosid mit einem zwischen den Positionen 4' und 5' ungesättigten Pyranose-Ring, welcher durch eine Aminoalkylgruppe in der 5'-Position substituiert ist, erhältlich durch
—Auflösen des antibackteriellen Aminoglycosids oder eines Salzes desselben in Wasser,
—Einstellen des pH-Wertes der Lösung auf 7,3 bis 7,4,
—Zugabe eines Antioxidans zu der Lösung,
—Hindurchperlen von Stickstoff durch die Lösung nach steriler Filtration sowie
—Abfüllen der so erhaltenen Lösung unter Stickstoff in Ampullen.

2. Pharmazeutische Zusammensetzungen nach Anspruch 1 erhältlich durch Verwendung von Sisomicin, Netylmicin oder 5-epi-Sisomicin oder deren parenteral akzeptablen Salze als antibackterielles Aminoglycosid.

3. Pharmazeutische Zusammensetzungen nach Anspruch 1 oder 2, erhältlich durch die Verwendung von Natriumphosphit oder Natriumsulfit als Antioxidans.

4. Pharmazeutische Zusammensetzungen nach einem der Ansprüche 1 bis 3, erhältlich durch Einstellung des pH-Werts der Lösung mittels eines Puffersystems aus 2-Amino-2-hydroxymethyl-1,3-propandiol.

5. Verfahren zur Herstellung der pH- und farbstabilisierten parenteralen pharmazeutischen Zusammensetzungen nach einem der Ansprüche 1 bis 4 gekennzeichnet durch
—Auflösen eines antibakteriellen Aminoglycosids mit einem Pyranose-Ring, der zwischen den 4'- und 5'-Positionen ungesättigt ist und durch eine Aminoalkylgruppe an der 5'-Position substituiert ist, oder eines pharmakologisch akzeptablen Salzes davon, in Wasser;
—Einstellen des pH-Werts der Lösung auf 7,3—7,4;
—Zugabe eines Antioxidans zu der Lösung;
—Hindurchperlen von Stickstoff durch die Lösung nach steriler Filtration; und
—Abfüllen der so erhaltenen Lösung in Ampullen unter Stickstoff.

6. Verfahren nach Anspruch 5 gekennzeichnet durch die Verwendung von Sisomicin, Netylmicin oder 5-epi-Sisomycin oder deren parenteral verträglichem Salz als antibakterielles Aminoglycosid.

7. Verfahren nach Anspruch 5 oder 6, gekennzeichnet durch die Verwendung von Natriumphosphit oder Natriumsulfit als Antioxidant.

8. Verfahren nach einem der Ansprüche 5 bis 7 gekennzeichnet durch die Einstellung des pH-Werts der Lösung mittels eines Puffersystems aus 2-Amino-2-oxymethyl-1,3-propandiol.

**Revendications**

1. Compositions pharmaceutiques parentérales aqueuses, à pH et coloration stabilisés, comprenant un aminoglycoside antibactérien comprenant un cycle pyranose qui est insaturé entre les positions 4' et 5' et qui est substitué par un groupe aminoalkyle en position 5', que l'on peut obtenir par:
—dissolution dans l'eau de l'aminoglycoside antibactérien ou d'un sel de celui-ci,
—ajustement du pH de la solution à 7,3—7,4,
—addition d'un antioxydant à la solution,
—barbotage d'azote à travers la solution après filtration stérile, et
—remplissage d'ampoules, sous azote, par la solution ainsi obtenue.

2. Compositions pharmaceutiques selon la revendication 1, que l'on peut obtenir en utilisant la sisomycine, la nitylmycine ou l'épi-5-sisomycine ou leurs sels parentéralement acceptables, en tant qu'aminoglycoside antibactérien.

3. Compositions pharmaceutiques selon l'une des revendications 1 ou 2, que l'on peut obtenir un utilisant du phosphite de sodium ou du sulfite de sodium en tant qu'antioxydant précité.

4. Compositions pharmaceutiques selon l'une des revendications 1 à 3, que l'on peut obtenir en ajustant le pH de la solution avec un système tampon comprenant de l'amino-2 hydroxyméthyl-2 propanediol-1,3.

5. Procédé de préparation de compositions pharmaceutiques parentérales aqueuses, à pH et coloration stabilisés, selon l'une des revendications 1 à 4, caractérisé par:

—la dissolution dans l'eau d'un aminoglycoside antibactérien comprenant un cycle pyranose qui est insaturé entre les positions 4' et 5' et qui est substitué par un groupe aminoalkyle en position 5' ou un sel pharmacologiquement acceptable de celui-ci,

—l'ajustement du pH de la solution à 7,3—7,4,

—l'addition d'un antioxydant à la solution,

—le barbotage d'azote à travers la solution après filtration stérile, et

—le remplissage d'ampoules, sous azote, par la solution ainsi obtenue.

6. Procédé selon la revendication 5, caractérisé par l'utilisation de la sisomycine, de la nitylmycine ou de l'épi-5-sisomycine ou de leurs sels parentéralement acceptables, en tant qu'aminoglycoside antibactérien.

7. Procédé selon l'une des revendications 5 ou 6, caractérisé par l'utilisation du phosphite de sodium ou du sulfite de sodium en tant qu'antioxydant précité.

8. Procédé selon l'une des revendications 5 à 7, caractérisé par l'ajustement du pH de la solution avec un système tampon comprenant de l'amino-2 hydroxyméthyl-2 propanediol-1,3.

EP 0 195 114 B1

Voltammogram of sisomicin sulphate solution prepared with Britton Robinson buffer of pH 4

Fig.1

Voltammogram of sisomicin sulphate solution prepared with Britton-Robin-son buffer of pH 6,2

Fig.2

Voltammogram of sisomicin sulphate solution prepared with Britton-Robinson buffer of pH 7,3

Fig.3